# EUROPEAN PATENT SPECIFICATION

(11) **EP 1 891 906 B1**
(45) Date of publication and mention of the grant of the patent: **22.05.2013**
(21) Application number: 05820068.4
(22) Date of filing: 20.12.2005
(51) Int. Cl.: A61B 18/14, A61B 18/12

(54) **MEDICAL ELECTRODE NEEDLE DEVICE**
MEDIZINISCHE ELEKTRODENNADELVORRICHTUNG
DISPOSITIF A AIGUILLE MEDICALE A ELECTRODE

(30) Priority: 22.06.2005 JP 2005181719
(43) Date of publication of application: 27.02.2008
(73) Proprietor: KABUSHIKI KAISHA TOP, Tokyo 120-0035 (JP)
(72) Inventor: KOBAYASHI, Susumu, c/o KABUSHIKI KAISHA TOP, Tokyo 120-0035 (JP); YAMADA, Hidehiko, c/o KABUSHIKI KAISHA TOP, Tokyo 120-0035 (JP); IIDA, Naoyuki, c/o KABUSHIKI KAISHA TOP, Tokyo 120-0035 (JP)
(74) Representative: Henkel, Breuer & Partner
(86) International application number: PCT/JP2005/023350
(87) International publication number: WO 2006/137184

(56) References cited:
- WO-A-00/66014
- WO-A-98/06341
- WO-A1-00/66014
- WO-A1-96/16606
- WO-A1-98/06341
- US-A- 5 542 915
- US-A- 6 045 549
- US-A- 6 045 549
- US-A1- 2003 209 264
- US-A1- 2003 209 264
- US-A1- 2004 193 152
- US-B1- 6 475 214
- US-B1- 6 475 214

## Description

### Technical Field

The present invention relates to a needle electrode device for medical application that is pierced into an affected area to perform therapeutic thermocoagulation by radiofrequency current.

### Background Art

Conventionally, there is already known a needle electrode device for thermally coagulating affected tissue by supplying radiofrequency current to a needle electrode pierced into the affected area of a living body (for example, refer to Japanese Unexamined PCT Publication No. 2002-532186).

This type of needle electrode device has a needle electrode whose tip is sharply formed and the needle electrode is supplied with radiofrequency current via a lead wire electrically connected to the needle electrode. With the tip placed on the affected area, the needle electrode heats the area around the tip of the needle electrode by the supplied radiofrequency current to thermally coagulate the affected tissue.

If the needle electrode generates the heat insufficiently or excessively when thermally coagulating the affected area by the radiofrequency current supplied to the needle electrode, an appropriate coagulative lesion cannot be obtained in the affected tissue.

Consequently, it is conceivable to detect a temperature of the heated part of the needle electrode with a thermocouple attached to the needle electrode and to control the heating time for the affected area and the supply of the radiofrequency current to the needle electrode while checking the temperature detected by the thermocouple. Moreover, the thermocouple can be formed extremely thin and therefore it is advantageous in that the needle electrode can be formed relatively compact even if it is attached along the needle electrode.

The thermocouple, however, has a disadvantage of being affected by the radiofrequency current supplied to the needle electrode and sometimes causing inclusion of a high frequency noise in a detected temperature signal generated by the thermocouple, which disables accurate temperature detection.

US2004/193152A1 discloses an electrosurgical device having a hollow shaft, an electrically conductive solid distal tip that is adapted for penetrating bone, and an intermediate electrically insulating spacer. Electrodes are disposed within a window extending transversely through the side wall of the hollow shaft. A thermocouple is disposed within the insulating spacer which is made for example of PTFE, nylon, an epoxy, a polyurethane, a polyimide or a ceramic material and is provided between an inner rim of the window and the electrodes.

WO00/66014A, WO98/06341A and US6045549A respectively disclose an apparatus for a thermal treatment of tissue having a hollow needle electrode and a thermocouple having its tip embedded in an epoxy sealant closing the tip end portion of the hollow needle electrode.

US2003/209264A1 discloses a thermocouple with a thermocouple junction at a distal end of first and second thermocouple conductors and a heat-shrinkable polymer material formed over the thermocouple junction and sealing the thermocouple junction. The purpose of this polymer material is to provide an insulated thermocouple device of extremely small size. The document mentions polyethylene terephthalate (PET) or fluorinated ethylene propylene (FEP) as suitable polymer material for the heat shrinking.

### DISCLOSURE OF THE INVENTION

It is an object of the present invention to provide a needle electrode device for medical application capable of being compact with a needle electrode provided with a thermocouple and of detecting a temperature using the thermocouple with high accuracy.

To achieve the above object the present invention provides a needle electrode device as defined in claim 1. According to the present invention, there is provided a needle electrode device for medical application having a needle electrode, which can be pierced into an affected area and is coated with an insulating coating material on the outer surface except the tip of the needle electrode, and adapted to supply the needle electrode with radiofrequency current, the needle electrode device comprising a thermocouple for detecting a temperature of the tip of the needle electrode, wherein the thermocouple includes a tip connecting portion where the tips of a pair of coated wires constituting the thermocouple are electrically connected to each other, and a tip coated portion where the tip connecting portion is coated with polyolefin synthetic resin, and wherein the tip coated portion is bonded and fixed to the needle electrode with adhesive.

According to the present invention, the temperature of the tip of the needle electrode is detected by the thermocouple provided. Therefore, the supply of the radiofrequency current can be controlled in such a way that the needle electrode reaches an appropriate heat temperature according to the temperature detected by the thermocouple.

While a thermocouple is composed of a pair of coated wires and the tips of both wires are electrically connected to each other through solder or the like with the tip coatings removed, the tip connecting portion is provided with the tip coated portion coated with the polyolefin synthetic resin (for example, polyethylene or polypropylene) in the present invention. Therefore, even if the thermocouple is provided in contact with the needle electrode of conductive metal, high frequency noise can be prevented from entering the detected temperature signal generated by the thermocouple, by which the temperature can be detected with high accuracy.

Moreover, only the tip coated portion of the thermocouple is bonded to the needle electrode with the adhesive in the present invention. Therefore, only a small amount of adhesive is consumed and the thermocouple can be bonded to the needle electrode very easily during manufacturing. Furthermore, the thermocouple bonded to the needle electrode detects the heat temperature of the needle electrode in the fixed position and therefore it can detect the temperature of the heated area without fail.

Moreover, according to the present invention, the needle electrode is formed hollow with the thermocouple contained in the needle electrode, the tip coated portion is bonded and fixed to a part of the inner surface of the needle electrode with adhesive, and the needle electrode has a gap therein formed between the thermocouple for freely conducting a drug solution over the entire length of the needle electrode.

With the formation of the hollow needle electrode, the drug solution can be injected into the affected area through the needle electrode. Moreover, the wires constituting the thermocouple can be formed extremely thin. Therefore, the hollow needle electrode can contain and support the thermocouple inside. This reduces a piercing resistance to an extremely low level when the needle electrode is pierced into the affected area of the living body in comparison with a case in which the thermocouple is provided outside the needle electrode. Moreover, the gap inside the needle electrode prevents the thermocouple from blocking up the inside hole of the needle electrode, so that the drug solution can be smoothly injected into the affected area through the needle electrode. In addition, the thermocouple is covered outside with the needle electrode. Therefore, it is possible to prevent the disconnection or the like of the wires constituting the thermocouple without fail even if the wires are extremely thin.

With these features, preferably the tip of the needle electrode is sharply formed with a beveled edge relative to the axis line of the needle electrode as a blade surface, and the tip coated portion of the thermocouple is bonded to a tip inner surface, which is exposed inside the blade surface of the needle electrode. Since the tip inner surface of the needle electrode is exposed inside the blade surface, the tip coated portion of the thermocouple is exposed by positioning the tip coated portion of the thermocouple in the tip inner surface of the needle electrode, by which the adhesive can be applied very easily during bonding of the tip coated portion. Moreover, the hardening of the adhesive applied to the tip inner surface of the needle electrode rapidly progresses, which leads to a high manufacturing efficiency. Furthermore, since the adhesive is applied only to the exposed tip inner surface of the needle electrode, the adhesive does not block up the inside hole of the needle electrode, thereby achieving the smooth conduction of the drug solution over the entire length of the needle electrode.

### Brief Description of the Drawings

FIG. 1 is an explanatory diagram showing the appearance of a needle electrode device according to the present invention.
FIG. 2 is a cross section schematic diagram of a substantial part according to a first embodiment of the present invention.
FIG. 3 is an explanatory enlarged cross-sectional view showing the bonded state of the tip of a thermocouple in the first embodiment.
FIG. 4 is a cross section schematic diagram of the tip of the thermocouple.
FIG. 5(a) is a chart showing a temperature detected by the thermocouple in the embodiment.
FIG. 5(b) is a chart showing a temperature detected by a conventional thermocouple.

### Best Mode for Carrying out the Invention

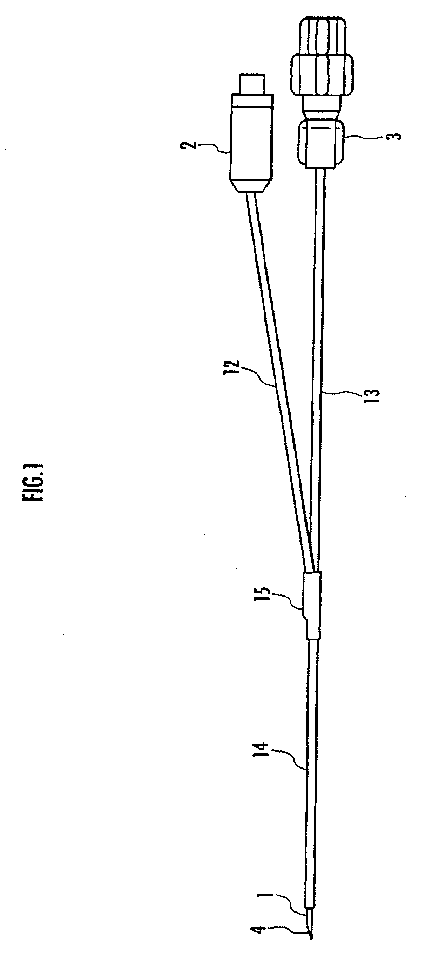

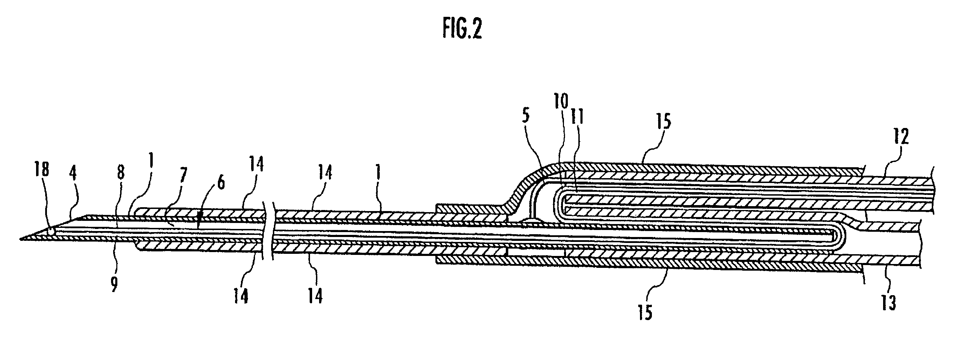

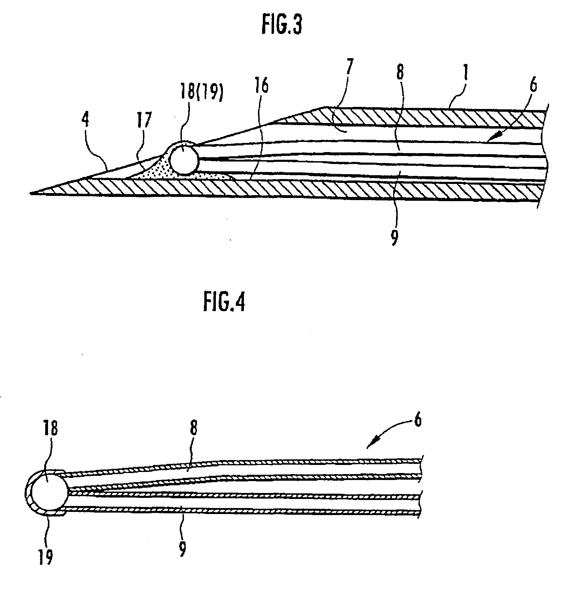

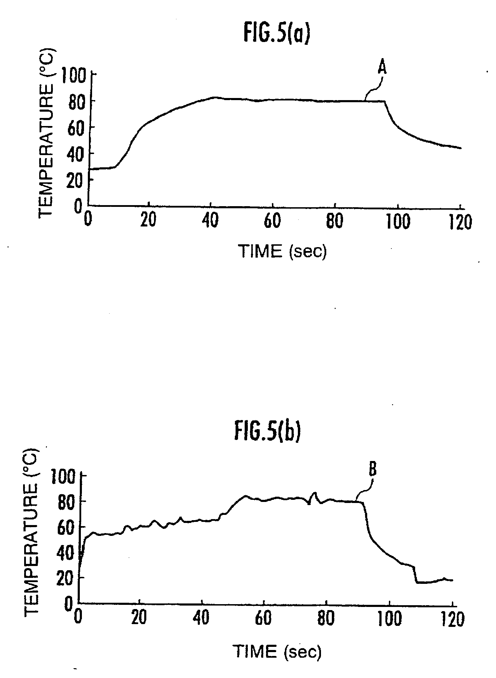

The present invention will be described below by using the accompanying drawings. FIG. 1 is an explanatory diagram showing a needle electrode device according to a first embodiment of the present invention. FIG. 2 is a cross section schematic diagram of a substantial part according to the first embodiment of the present invention. FIG. 3 is an explanatory enlarged cross-sectional view showing the bonded state of the tip of a thermocouple in the first embodiment. FIG. 4 is a cross section schematic diagram of the tip of the thermocouple. FIG. 5 is a chart showing a temperature detected by the thermocouple. FIG. 6 is a cross section schematic diagram of a substantial part according to a second embodiment of the present invention. FIG. 7 is an explanatory enlarged cross-sectional view showing the bonded state of the tip of a thermocouple in the second embodiment.

As shown in FIG. 1, the needle electrode device according to the first embodiment includes a needle electrode 1 to be pierced into an affected area of a living body, a first connector 2 for a connection between a feeder line and a signal line, and a second connector 3 for injecting a drug solution. The first connector 2 connects to a high frequency power source and temperature measurement means, which are not shown. The second connector 3 connects to drug solution injection means, which is not shown.

Describing the needle electrode device according to the first embodiment more specifically, as shown in FIG. 2, the needle electrode 1 is formed into a hollow stainless or other metal component having electrical conductivity and the tip of the needle electrode 1 is sharply formed with a beveled edge relative to the axis line of the needle electrode 1 as a blade surface 4.

Furthermore, a feeder line 5 is electrically connected to the outer surface of the bottom of the needle electrode 1 with solder and the needle electrode 1 contains a thermocouple 6 inside. A gap 7 is provided between the inner surface of the needle electrode 1 and the thermocouple 6 and the gap 7 is used as a drug solution flow passage. The thermocouple 6 used in this embodiment is composed of a pair of coated wires 8 and 9, which are a chromel wire and an alumel wire, and signal lines 10 and 11 continuing from the wires 8 and 9 are extending rearward with being contained in a wire tube 12 together with the feeder line 5. The bottom of the needle electrode 1 is connected to a drug solution introduction tube 13.

Moreover, the needle electrode 1 is coated with a first coating material 14 having insulation properties (insulating coating material) on its outer surface except the tip of the needle electrode 1. At the bottom of the needle electrode 1, one end of the wire tube 12 and one end of the drug solution introduction tube 13 are tied together and coated with a second coating material 15. As shown in FIG. 1, the first connector 2 is attached to the other end of the wire tube 12 and the second connector 3 is attached to the other end of the drug solution introduction tube 13.

As shown in FIG. 3, the tip of the thermocouple 6 is bonded to a tip inner surface 16 exposed inside the blade surface 4 of the needle electrode 1 with adhesive 17. At the tip of the thermocouple 6 in the bonded region, there is formed a tip connecting portion 18 where the tips of the wires 8 and 9 constituting the thermocouple 6 are electrically connected to each other by welding as shown in FIG. 4. Furthermore, the tip connecting portion 18 is provided with a tip coated portion 19 coated with polyolefin synthetic resin such as polyethylene or polypropylene.

According to this embodiment, the tip of the thermocouple 6 is bonded to the tip inner surface 16 exposed inside the blade surface 4 of the needle electrode 1. Therefore, the bonding operation is quite easy, which leads to a high manufacturing efficiency. Furthermore, the tip coated portion 19 is provided at the tip of the thermocouple 6 in the bonded region, which prevents high frequency noise from entering a detected temperature signal generated by the thermocouple. More specifically, the present inventor has performed a comparison test between the tip of the thermocouple 6 provided with the tip coated portion 19 (corresponding to this embodiment) and the tip of the thermocouple 6 without the tip coated portion 19. In this test, the output of the high frequency power source is previously adjusted in such a way as to achieve an 80°C temperature at the tip of the thermocouple 6 to measure a temperature detected by the thermocouple 6. Consequently, as shown in FIG. 5(a), it is found that line A representing the detected temperature signal is relatively smooth and stable temperatures are observed during a predetermined time period after the rise up regarding the tip of the thermocouple 6 with the tip coated portion 19 provided. On the other hand, regarding the tip of the thermocouple 6 without the tip coated portion 19, as shown in FIG. 5(b), line B representing the detected temperature signal is not smooth and the inclusion of high frequency noise has been observed in the detected temperature signal of the thermocouple 6, which leads to a low detection accuracy. As described hereinabove, according to this embodiment, the inclusion of the high frequency noise can be prevented by providing the tip coated portion 19 at the tip of the thermocouple 6, and therefore the temperature can be detected with high accuracy.

Still further, the thermocouple 6 is contained in the needle electrode 1 and thus it is covered outside with and protected by the needle electrode 1. Therefore, the disconnection or the like of the wires 8 and 9 can be reliably prevented even if the wires 8 and 9 of the thermocouple 6 are formed very thin.

### Industrial Applicability

As described hereinabove, according to the present invention, the needle electrode is provided with the thermocouple in a compact form and the thermocouple can detect a temperature very accurately. Therefore, the present invention can be preferably used as a needle electrode device for thermally coagulating an affected tissue by supplying radiofrequency current to a needle electrode pierced into the affected area of a living body.

## Claims

1. A needle electrode device for medical application having a needle electrode (1), which can be pierced into an affected area and is coated with an insulating coating material (14) on an outer surface except a tip of the needle electrode (1), and which is adapted to be supplied with radiofrequency current, the needle electrode device comprising:
a thermocouple (6) for detecting a temperature of the tip of the needle electrode (1),
wherein the thermocouple (6) includes a tip connecting portion (18) where tips of a pair of coated wires (8,9) constituting the thermocouple (6) are electrically connected to each other, and a tip coated portion (19) where the tip connecting portion (18) is coated with polyolefin synthetic resin,
wherein the tip coated portion (19) is bonded and fixed to the needle electrode (1) with adhesive (17), and
wherein the needle electrode (1) is formed hollow with the thermocouple (6) contained in the needle electrode (1), the tip coated portion (19) is bonded and fixed to a part of an inner surface of the needle electrode (1) with the adhesive (17), and the needle electrode (1) has a gap (7) therein formed between the thermocouple (6) for freely conducting a drug solution over the entire length of the needle electrode (1).

2. A needle electrode device for medical application according to claim 1, wherein the tip of the needle electrode (1) is sharply formed with a beveled edge relative to the axis line of the needle electrode (1) as a blade surface (4), and wherein the tip coated portion (19) of the thermocouple (6) is bonded to a tip inner surface (16), which is exposed inside the blade surface (4) of the needle electrode (1).

## Patentansprüche

1. Eine Elektrodennadelvorrichtung zur medizinischen Anwendung mit einer Elektrodennadel (1), welche in einen betroffenen Bereich eingestochen werden kann und mit einem isolierenden Beschichtungsmaterial (14) auf einer Außenfläche mit Ausnahme einer Spitze der Elektrodennadel (1) beschichtet ist, und welche dazu eingerichtet ist, mit Hochfrequenzstrom versorgt zu werden, wobei die Elektrodennadelvorrichtung umfasst:
ein Thermoelement (6) zum Erfassen einer Temperatur der Spitze der Elektrodennadel (1),
wobei das Thermoelement (6) einen Endenverbindungsabschnitt (18), wo Enden eines Paares von beschichteten Drähten (8,9), welche das Thermoelement (6) bilden, miteinander elektrisch verbunden sind, und einen beschichteten Endabschnitt (19), wo der Endenverbindungsabschnitt (18) mit synthetischem Polyolefinharz beschichtet ist, umfasst,
wobei der beschichtete Endabschnitt (19) mit der Elektrodennadel (1) mit Klebstoff (17) verklebt und daran fixiert ist, und
wobei die Elektrodennadel (1) hohl ausgebildet ist und das Thermoelement (6) in der Elektrodennadel (1) enthalten ist, der beschichtete Endabschnitt (19) an einem Teil einer Innenfläche der Elektrodennadel (1) mit dem Klebstoff (17) verklebt und daran fixiert ist, und die Elektrodennadel (1) einen Zwischenraum (7) aufweist, welcher in ihr zwischen dem Thermoelement (6) zum freien Leiten einer Arzneimittellösung über der gesamten Länge der Elektrodennadel (1) ausgebildet ist.

2. Eine Elektrodennadelvorrichtung zur medizinischen Anwendung gemäß Anspruch 1, wobei die Spitze der Elektrodennadel (1) scharf mit einem abgeschrägten Rand relativ zu der Axiallinie der Elektrodennadel (1) als eine Schneidfläche (4) ausgebildet ist, und wobei der beschichtete Endabschnitt (19) des Thermoelements (6) mit einer Spitzenoberfläche (16), welche innerhalb der Schneidfläche (4) der Elektrodennadel (1) freiliegend ausgebildet ist, verklebt ist.

## Revendications

1. Dispositif à électrode-aiguille pour une application médicale comportant une électrode-aiguille (1), qui peut être percée dans une zone affectée et qui est revêtue d'un matériau de revêtement isolant (14) sur une surface extérieure, à l'exception d'une pointe de l'électrode-aiguille (1), et qui est conçue pour être alimentée en courant radiofréquence, le dispositif à électrode-aiguille comprenant :
un thermocouple (6) pour détecter une température de la pointe de l'électrode-aiguille (1),
dans lequel le thermocouple (6) comprend une partie de connexion de pointes (18) où les pointes d'une paire de fils revêtus (8, 9) constituant le thermocouple (6) sont connectées électriquement l'une à l'autre, et une partie revêtue de pointe (19) où la partie de connexion de pointes (18) est revêtue d'une résine synthétique polyoléfinique,
dans lequel la partie revêtue de pointe (19) est collée et fixée à l'électrode-aiguille (1) par un adhésif (17), et
dans lequel l'électrode-aiguille (1) a une forme creuse, le thermocouple (6) étant contenu dans l'électrode-aiguille (1), la partie revêtue de pointe (19) est collée et fixée à une partie d'une surface intérieure de l'électrode-aiguille (1) par l'adhésif (17), et l'électrode-aiguille (1) comporte un espace (7) dans celle-ci formé entre le thermocouple (6) pour conduire librement une solution médicamenteuse sur la longueur entière de l'électrode-aiguille (1).

2. Dispositif à électrode-aiguille pour une application médicale selon la revendication 1, dans lequel la pointe de l'électrode-aiguille (1) a une forme pointue avec un bord biseauté par rapport à la ligne d'axe de l'électrode-aiguille (1) en tant que surface de lame (4), et dans lequel la partie revêtue de pointe (19) du thermocouple (6) est collée à une surface intérieure de pointe (16), qui est exposée à l'intérieur de la surface de lame (4) de l'électrode-aiguille (1).
